(19) Europäisches Patentamt
European Patent Office
Office européen des brevets

(11) **EP 3 500 338 B1**

(12) **EUROPÄISCHE PATENTSCHRIFT**

(45) Veröffentlichungstag und Bekanntmachung des Hinweises auf die Patenterteilung:
**09.11.2022   Patentblatt 2022/45**

(21) Anmeldenummer: **17765353.2**

(22) Anmeldetag: **16.08.2017**

(51) Internationale Patentklassifikation (IPC):
***A61N 1/08*** *(2006.01)*

(52) Gemeinsame Patentklassifikation (CPC):
**A61N 1/08**

(86) Internationale Anmeldenummer:
**PCT/EP2017/070790**

(87) Internationale Veröffentlichungsnummer:
**WO 2018/033583 (22.02.2018 Gazette 2018/08)**

(54) **VORRICHTUNG UND VERFAHREN ZUR PRÜFUNG DER MR-SICHERHEIT VON IMPLANTATEN**

APPARATUS AND METHOD FOR TESTING THE MR SAFETY OF IMPLANTS

DISPOSITIF ET PROCÉDÉ POUR TESTER LA SÉCURITÉ À LA RÉSONANCE MAGNÉTIQUE D'IMPLANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.08.2016   DE 102016115216**

(43) Veröffentlichungstag der Anmeldung:
**26.06.2019   Patentblatt 2019/26**

(73) Patentinhaber: **MR COMP GmbH
45894 Gelsenkirchen (DE)**

(72) Erfinder: **SCHAEFERS, Gregor
46236 Bottrop (DE)**

(74) Vertreter: **Schneiders & Behrendt Bochum
Huestraße 23
44787 Bochum (DE)**

(56) Entgegenhaltungen:
**US-A1- 2010 253 338     US-A1- 2011 066 028**

- **SUKHOON OH ET AL: "Experimental and numerical assessment of MRI-induced temperature change and SAR distributions in phantoms and in vivo", MAGNETIC RESONANCE IN MEDICINE., Bd. 63, Nr. 1, 1. Januar 2010 (2010-01-01), Seiten 218-223, XP055398491, US ISSN: 0740-3194, DOI: 10.1002/mrm.22174**
- **Anonymous: "Birdcage RF coil - Questions and Answers​in MRI", , 11. August 2016 (2016-08-11), XP055433002, Gefunden im Internet: URL:https://web.archive.org/web/2016081118 3314/http://mriquestions.com:80/birdcage-c oil.html [gefunden am 2017-12-07]**

## Beschreibung

Die bildgebende medizinische Diagnose mittels magnetischer Resonanz (MR), auch als Magnetresonanztomographie (MRT) oder "Magnetic Resonance Imaging" (MRI) bezeichnet, ist weit verbreitet. Zur Erzeugung von messbaren MR-Signalen wird in den verwendeten MR-Systemen der Körper des untersuchten Patienten mit hochfrequenter (HF) elektromagnetischer Strahlung (bei Frequenzen im Bereich zwischen 5 MHz und mehreren 100 MHz) hoher Leistung (mehrere 100 W bis mehrere kW) beaufschlagt. Die HF-Strahlung wird im Körpergewebe zum Teil absorbiert und bewirkt dort während der MR-Untersuchung eine Erwärmung. Insbesondere wird die HF-Strahlung in Metall enthaltenden medizinischen Implantaten, die sich im Körper befinden, absorbiert. Diese erwärmen sich besonders stark und können daher im umgebenden Gewebe zu Verletzungen führen und somit ein Sicherheitsrisiko darstellen.

Es ist somit von Bedeutung, die Sicherheit eines bestimmten Implantates für MR-Untersuchungen (MR-Sicherheit) vorab festzustellen, d.h. bevor ein Patient, der ein solches Implantat trägt, einer MR-Untersuchung unterzogen wird.

Die Veröffentlichung "Sukhoon Oh et. Al." ("Experimental and numerical Assessment of MRI-Induced Temperature Change and SAR Distributions in Phantoms and In Vivo", Magnetic Recsonance in Medicine 63:218-223 (2010)) beschreibt eine Vorrichtung, bei der ein Testvolumen, in dem eine Probe angeordnet ist, in einem röhrenförmigen Magnetresonanztomographie-Vorrichtung (MRT) angeordnet ist und mittels einer röhrenförmigen Spule als HF-Sendevorrichtung mit hochfrequenter elektromagnetischer Strahlung beaufschlagt wird.

Die Veröffentlichung US 2010/253338 A1 beschreibt eine weitere Vorrichtung zur Prüfung der MR-Sicherheit von Implantaten.

Die Messung der Erwärmung des Implantates oder des dieses umgebenden Gewebes oder auch der HF-Absorption im/am Implantat, die zu einer entsprechenden Erwärmung führt, in vivo ist schwierig.

Es besteht daher Bedarf nach Vorrichtungen und Verfahren, die eine gefahrlose und zuverlässige Überprüfung der MR-Sicherheit eines Implantates außerhalb des Körpers ermöglichen.

Hierzu schlägt die Erfindung eine Vorrichtung zur Prüfung der MR-Sicherheit eines Implantates vor, mit einem Phantom, das ein mit einem Medium gefülltes Testvolumen umfasst, wobei das Testvolumen das zu prüfende Implantat aufnimmt, wenigstens einer HF-Sendevorrichtung, die hochfrequente elektromagnetische Strahlung in das Testvolumen hinein emittiert, und mit Mitteln zur Erfassung der Erwärmung des Implantates und/oder des dieses umgebenden Mediums. Davon sind auch Mittel zur Erfassung von elektromagnetischen Messgrößen (z.B. absorbierte Strahlungsleistung) umfasst, die auf die Erwärmung zurückschließen lassen.

Das Medium des Testvolumens kann eine Flüssigkeit, ein Gel, Luft oder eine Mischung aus diesen Materialien sein.

Erfindungsgemäß wird also das zu überprüfende Implantat in das Testvolumen des Phantoms eingebracht und dort mit HF-Strahlung beaufschlagt, wobei die Situation einer MR-Untersuchung nachgebildet wird. Dabei wird die Erwärmung des Implantates oder des dieses umgebenden Mediums gemessen, z.B. mittels Temperatursensoren, die sich ebenfalls in dem Testvolumen, vorzugsweise in dem Medium in der Nähe des Implantates befinden, oder auch kontaktlos z.B. mittels geeigneter optischer Faserthermometer oder mittels sonstiger Strahlungsthermometer. Die Erwärmung kann auch (indirekt) über Sensoren zur Erfassung der elektrischen und/oder magnetischen Feldstärke ermittelt werden oder mittels andersartiger Sensorik.

Die Erfindung schlägt außerdem ein Verfahren zur Prüfung der MR-Sicherheit eines Implantates vor, wobei ein mit einem Medium gefülltes Testvolumen das zu prüfende Implantat aufnimmt und mittels wenigstens einer HF-Sendevorrichtung mit hochfrequenter elektromagnetischer Strahlung beaufschlagt wird, wobei die Erwärmung des Implantates und/oder des dieses umgebenden Mediums erfasst wird. Davon ist auch die Erfassung der Veränderung der elektromagnetischen Felder in Anwesenheit des Implantates umfasst, aus der auf die Erwärmung zurückgeschlossen werden kann.

Bei der erfindungsgemäßen Vorrichtung umfasst die HF-Sendevorrichtung zudem eine Anordnung aus mehreren Antennenelementen, wobei jedes Antennenelement mit einem HF-Signal mit einer für dieses Antennenelement individuell vorgebbaren Amplitude und/oder Phase beaufschlagbar ist. Die Anordnung aus mehreren Antennenelementen bildet ein Antennenarray, dessen einzelne Elemente gezielt jeweils mit einem HF-Signal individuell einstellbarer Amplitude und/oder Phase beaufschlagt werden, um so nahezu jede beliebige HF-Feldverteilung in dem Testvolumen, insbesondere am Ort des zu prüfenden Implantates, variabel einstellen zu können. Es geht dabei darum, die Situation bei einer realen MR-Untersuchung so gut wie möglich nachzubilden. Weiter geht es darum, die gewissermaßen "Worst-Case"-Situation aufzufinden, d.h. diejenige elektromagnetische Feldverteilung, bei der die HF-Absorption und damit die Erwärmung maximal wird. Dieser Worst Case, das heißt die maximal mögliche Erwärmung des Implantates, ist das entscheidende Kriterium für die Beurteilung des Implantates als sicher oder unsicher für MR-Untersuchungen. Vor diesem Hintergrund wird zweckmäßig die Erwärmung für verschiedene eingestellte Feldverteilungen ermittelt, und diejenige Feldverteilung für die Bestimmung der MR-Sicherheit herangezogen, bei der die Erwärmung maximal ist. Das Implantat wird als MR-sicher (oder bedingt MR-sicher) charakterisiert, wenn die maximale Erwärmung unter einem vorgegebenen Grenzwert liegt.

Hierzu sind zweckmäßigerweise die Antennenelemente mit einem mehrkanaligen HF-Sendeverstärker verbunden, wobei Amplitude und/oder Phase in jedem Kanal individuell einstellbar sind. Der HF-Sendeverstärker kann beispielsweise aus einem HF-Synthesizer bestehen, dessen Ausgangssignal auf mehrere Kanäle aufgeteilt wird. In jedem Kanal befinden sich dabei ein variabler Abschwächer zur Einstellung der Amplitude des HF-Signals sowie ein Phasenschieber zur Einstellung der Phase des HF-Signals in diesem Kanal. In jedem Kanal ist ein HF-Verstärker vorgesehen, um die für das jeweilige Antennenelement benötigte HF-Leistung zur Verfügung zu stellen.

Bei einer besonders bevorzugten Ausgestaltung sind die Antennenelemente Streifenleitungen, die parallel zu wenigstens einer Oberfläche des Testvolumens verlaufen. Streifenleitungen sind dielektrische Wellenleiter, die aus einem oder mehreren dünnen, leitfähigen Streifen bestehen, die auf einem Dielektrikum aufgebracht sind oder in der Nähe eines Dielektrikums verlaufen. Streifenleiter können z.B. aus in einer Ebene angeordneten schmalen Leitungsstreifen bestehen, die isoliert in oder über einer metallischen Fläche angeordnet sind. Mit Streifenleitungen lassen sich kostengünstig und reproduzierbar Antennenstrukturen mit definierten Impedanzen herstellen.

Bevorzugt sind die Antennenelemente parallel zueinander verlaufende, geradlinige Streifenleitungen. Auf diese Weise lässt sich ein Array aus Antennen bilden (z.B. jeweils mit einer Dipol-artigen Charakteristik), mit dem sich in dem Testvolumen durch entsprechende Vorgabe der Amplituden und Phasen der HF-Signale nahezu jede beliebige HF-Feldverteilung erzeugen lässt. Dabei können die Streifenleitungen direkt auf die Oberfläche des Testvolumens oder auf eine das Testvolumen umgrenzende Wandung aufgebracht werden. Dies macht die erfindungsgemäße Vorrichtung besonders einfach und kostengünstig und führt zu einer hohen Reproduzierbarkeit bei der HF-Erzeugung. Auch lässt sich so eine stabile Anpassung der Antennenelemente erreichen. Die erzeugte elektromagnetische Strahlung wird direkt in das Medium, mit dem das Testvolumen gefüllt ist, eingekoppelt, d.h. ohne Verluste an Grenzflächen zwischen verschiedenen Medien.

In der Praxis hat es sich als zweckmäßig herausgestellt, ein Testvolumen mit einer quaderförmigen Geometrie zu verwenden, wobei die Streifenleitungen der Antennenanordnung im Wesentlichen quer zur Richtung der Längserstreckung des Quaders verlaufen. Dies ermöglicht es, eine elektromagnetische Feldverteilung im Inneren des Testvolumens zu erzeugen, bei der die elektrische Feldkomponente im Wesentlichen parallel zur Längserstreckung des Testvolumens verläuft. Die HF-Absorption im Implantat ist maximal, wenn die Richtung des elektromagnetischen Feldes parallel zur Oberfläche des Implantates verläuft. Um die maximale Erwärmung zu untersuchen, kann bei dieser Ausgestaltung entsprechend das Implantat einfach parallel zur Längserstreckung des Testvolumens ausgerichtet werden. Dies ist insbesondere bei länglichen Implantaten, beispielsweise bei drahtförmigen Implantaten, wie Kathetern oder Elektroden, von Bedeutung. Prinzipiell kann das Implantat in jeder gewünschten Orientierung in das Testvolumen eingebracht werden, beispielsweise ein drahtförmiges Implantat entlang einer spezifischen Trajektorie oder unter bestimmten Winkeln.

Um die realen Bedingungen im Körpergewebe nachzubilden, sollte das Medium, mit dem das Testvolumen des Phantoms gefüllt ist, hinsichtlich der elektrischen Permittivität und der elektrischen Leitfähigkeit biologischem Gewebe so weit wie möglich entsprechen. Auch sollten die thermische Leitfähigkeit und die Dichte des Mediums biologischem Gewebe annähernd entsprechen, damit die auftretende Wärmedissipation den realen Bedingungen nahekommt. Bei Implantaten, die im Gewebe und auch (teilweise) in Luft verbleibend implantiert werden, sollte das Medium entsprechend die elektromagnetischen Eigenschaften von Luft haben bzw. diesen nahekommen. Das Medium kann, wie erwähnt, Luft sein.

Bei dem erfindungsgemäßen Verfahren ist es nicht zwingend nötig, Messungen an einem realen Phantom mit darin befindlichem realem Implantat durchzuführen. Die Erwärmung des Implantates und/oder des dieses umgebenden Mediums kann auch durch eine numerische Simulation der Ausbreitung der elektromagnetischen Strahlung in dem Testvolumen und der Absorption der elektromagnetischen Strahlung in dem Implantat erfasst werden. Algorithmen zur numerischen Simulation elektromagnetischer Feldverteilungen, die durch bestimmte Antennenanordnungen erzeugt werden, sind aus dem Stand der Technik bekannt. Derartige Simulationsverfahren können erfindungsgemäß problemlos eingesetzt werden. Zu verweisen ist z.B. auf den Artikel von Z. Chen et al. in IEEE Transactions on Microwave Theory and Techniques, Band 64, Nr. 3, Seiten 972-981.

Ausführungsbeispiele der Erfindung werden nachfolgend anhand der Zeichnung näher erläutert. Es zeigt:

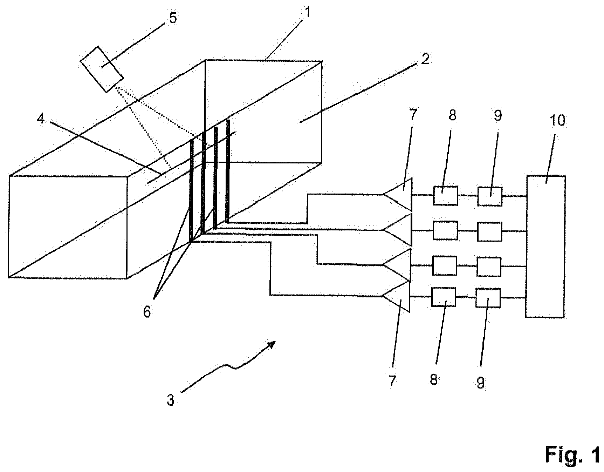

Figur 1:    schematische Ansicht einer erfindungsgemäßen Vorrichtung zur Prüfung der MR-Sicherheit eines Implantates.

In der Figur 1 ist eine erfindungsgemäße Vorrichtung schematisch dargestellt. Diese umfasst ein Phantom 1 in Form eines quaderförmigen Behältnisses, das ein Testvolumen im Sinne der Erfindung bildet. Die Dimensionen des Behältnisses betragen z.B. 600 x 600 x 2400 mm. Das Testvolumen ist mit einem Medium 2 gefüllt, das hinsichtlich elektrischer Permittivität und Leitfähigkeit biologischem Gewebe nahe kommt (z.B. $\varepsilon_r = 78$ und $\sigma = 0.47$). Weiterhin umfasst die dargestellte Vorrichtung eine HF-Sendevorrichtung, die insgesamt mit der Bezugsziffer 3 bezeichnet ist. Diese dient dazu, hochfrequente elektromagnetische Strahlung in das Testvolumen des Phantoms 1 hinein zu emittieren.

Die Frequenz der elektromagnetischen Strahlung liegt dabei im Bereich typischer MR-Frequenzen, d.h. im Bereich zwischen 5 Hz und einigen 100 MHz. Das Testvolumen des Phantoms 1 nimmt ein zu prüfendes medizinisches Implantat 4 auf. Bei dem dargestellten Ausführungsbeispiel ist das Implantat langgestreckt, z.B. drahtförmig. Jedes andere Implantat kann aber prinzipiell mit der dargestellten Vorrichtung ebenso geprüft werden. Weiterhin ist als Mittel zur Erfassung der Erwärmung des Implantates 4 und oder des dieses umgebenden Mediums 2 ein optisches Faserthermometer oder Strahlungsthermometer 5 vorgesehen, das die Temperatur an der Oberfläche des Implantates 4 misst. Andere Arten von Sensoren, wie z.B. im Medium angeordnete lokale Temperatursensoren können auch verwendet werden, wobei diese jedoch ggf. das elektromagnetische Feld stören. Auch kann die Erwärmung indirekt über Sensoren bestimmt werden, die das elektrische und/oder magnetische Feld in der Umgebung des Implantates 4 erfassen. Die HF-Sendevorrichtung 3 umfasst zur Erzeugung der gewünschten Feldverteilung eine Anordnung aus mehreren Antennenelementen 6. Es kann sich um geradlinige Streifenleitungen handeln, die parallel zueinander verlaufen. Anders designte Antennenelemente sind ebenfalls verwendbar. Die Antennenelemente 6 sind direkt auf die Oberfläche des das Phantom 1 bildenden Behältnisses aufgebracht sind. Bei dem Ausführungsbeispiel sind die Antennenelemente 6 auf nur eine der insgesamt sechs Wandungen des Behältnisses aufgebracht. Die in diesem Fall als Streifenleitungen ausgebildeten Antennenelemente 6 verlaufen quer zur Längserstreckung des Testvolumens. Die Einkopplung der Strahlung nur von einer Seite her in das Testvolumen reicht aus, um erfindungsgemäß verschiedene Feldverteilungen im Nahfeld der Antennenelemente 6 zur Prüfung der MR-Sicherheit zu erzeugen. Jedes Antennenelement 6 ist mittels der dargestellten HF-Sendevorrichtung 3 mit einem HF-Signal mit einer für dieses Antennenelement 6 individuell vorgebbaren Amplitude und Phase beaufschlagbar. Bei dem dargestellten Ausführungsbeispiel sind vier Antennenelemente 6 vorgesehen. Jedes der Antennenelemente 6 ist an den Ausgang eines HF-Sendeverstärkers 7 angeschlossen. Jedem HF-Sendeverstärker 7 sind ein einstellbarer Phasenschieber 8 sowie ein einstellbarer Abschwächer 9 vorgeschaltet. An ihren Eingängen sind die Abschwächer 9 mit einem HF-Synthesizer 10 verbunden, der an jedem Ausgang ein phasenstabiles HF-Signal bei der gewünschten Frequenz erzeugt

Die Erfindung erfordert allerdings nicht zwingend, reale Messungen an einem realen Phantom mit darin befindlichem realem Implantat durchzuführen. Die Erwärmung des Implantates und/oder des dieses umgebenden Mediums kann auch durch eine numerische Simulation der Ausbreitung der elektromagnetischen Strahlung in dem Testvolumen und der Absorption der elektromagnetischen Strahlung in dem Implantat erfasst werden. Hierfür können bekannte Verfahren zur numerischen Simulation der elektromagnetischen Feldverteilung bei der in Figur 1 dargestellten Anordnung verwenden werden. Die Deposition der HF-Leistung aufgrund der Anwesenheit des Implantates 4 ist wie folgt zu berechnen:

$$p = \int^{V}(p_{implant}(v) - p_{empty}(v)) \cdot dV \quad ,$$

Wobei $p_{implant}(v)$ die HF-Verlustleistungsdichte in Anwesenheit des Implantates und $p_{empty}(v)$ die HF-Verlustleistungsdichte ohne das Implantat ist. $V$ ist das relevante Volumen (auch als "hotspot integration volume" oder HSIV bezeichnet). Das HSIV ist definiert als ein zusammenhängendes Volumen, in dem $p_{implant}(v)$ signifikant größer ist als $p_{empty}(v)$.

Als Maß für das HF-Feld wird näherungsweise das tangentiale elektrische Feld $E_{tan}(z)$ verwendet, das ohne im Phantom 1 befindliches Implantat 4 entlang der Trajektorie (Längserstreckung) des Implantates 4 erzeugt wird. Die Position entlang der Trajektorie des Implantates 4 der Gesamtlänge $L$ (z.B. Drahtlänge) wird als z bezeichnet. Die Berechnung der HF-Verlustleistungsdichte aufgrund des Implantates 4 gemäß $E_{tan}(z)$ kann auf ein Modell des Implantates 4 gestützt werden, das einen Kalibrierfaktor A und eine Transferfunktion $S(z)$ vorsieht, die für das jeweilige Implantat (ggf. empirisch) zu bestimmen sind. Die Leistungsdeposition durch das Implantat kann dann einfach berechnet werden als:

$$p = A \cdot \left| \int_{0}^{L} S(z) \cdot E_{tan}(z) \cdot dz \right|^{2}$$

Die in-vivo HF-Leistungsdeposition wird auf diese Weise für verschiedene mittels der Antennenanordnung erzeugte Feldverteilungen $E_{tan}(z)$ berechnet. Die erhaltene HF-Verlustleistungsdichte kann dann in eine Temperaturänderung, d.h. eine lokale Erwärmung am Ort des Implantats 4 umgerechnet werden. Das Implantat 4 ist als sicher zu beurteilen, wenn der Temperaturanstieg unterhalb eines vorgegebenen Grenzwertes liegt.

**Patentansprüche**

1. Vorrichtung zur Prüfung der MR-Sicherheit eines Implantates (4), mit einem Phantom (1), das ein mit einem Medium (2) gefülltes Testvolumen umfasst, wobei das Testvolumen das zu prüfende Implantat (4) aufnimmt, wenigstens einer HF-Sendevorrichtung (3), die hochfrequente elektromagnetische Strahlung in das Testvolumen hinein emittiert, und mit Mitteln (5) zur Erfassung der Erwärmung des Implantates (4) und/oder des dieses umgebenden Mediums (2), wobei die HF-Sendevorrichtung (3) eine Anordnung aus mehreren Antennenelementen (6) umfasst, wobei jedes Antennenelement (6) mit einem HF-Signal mit einer für dieses Antennenelement (6) individuell vorgebbaren Amplitude und/oder Phase beaufschlagbar ist und wobei die Antennenelemente (6) auf eine das Testvolumen umgrenzende Wandung aufgebracht sind.

2. Vorrichtung nach Anspruch 1, wobei die Antennenelemente (6) mit einem mehrkanaligen HF-Sendeverstärker verbunden sind, wobei Amplitude und/oder Phase in jedem Kanal individuell einstellbar sind.

3. Vorrichtung nach Anspruch 1 oder 2, wobei die Antennenelemente (6) Streifenleitungen sind, die parallel zu wenigstens einer Oberfläche des Testvolumens verlaufen.

4. Vorrichtung nach Anspruch 4, wobei die Antennenelemente (6) parallel zueinander verlaufende, geradlinige Streifenleitungen sind.

5. Vorrichtung nach einem der Ansprüche 1 bis 4, wobei das Testvolumen eine quaderförmige Geometrie aufweist, wobei die als Streifenleitungen ausgebildeten Antennenelemente (6) im Wesentlichen quer zur Längserstreckung des Quaders verlaufen.

6. Vorrichtung nach einem der Ansprüche 1 bis 5, wobei das Medium hinsichtlich elektrischer Permittivität und Leitfähigkeit biologischem Gewebe entspricht.

7. Vorrichtung nach einem der Ansprüche 1 bis 6, wobei das Medium eine Flüssigkeit oder ein Gel oder Luft ist.

8. Verfahren zur Prüfung der MR-Sicherheit eines Implantates (4), bei dem eine Vorrichtung nach einem der Ansprüche 1 bis 7 verwendet wird, wobei das mit einem Medium (2) gefüllte Testvolumen das zu prüfende Implantat (4) aufnimmt und mittels der HF-Sendevorrichtung (3) mit hochfrequenter elektromagnetischer Strahlung beaufschlagt wird, wobei die Erwärmung des Implantates (4) und/oder des dieses umgebenden Mediums (2) erfasst wird.

9. Verfahren nach Anspruch 8, wobei jedes Antennenelement (6) mit einem HF-Signal mit einer für dieses Antennenelement (6) individuell vorgegeben Amplitude und/oder Phase beaufschlagt wird, in der Weise, das am Ort des Implantates (4) ein elektromagnetisches Feld erzeugt wird, das demjenigen elektromagnetischen Feld entspricht, das während einer MR-Untersuchung im Körper eines Patienten erzeugt wird.

10. Verfahren nach einem der Ansprüche 8 oder 9, wobei die Beaufschlagung mit elektromagnetischer Strahlung in der Weise erfolgt, dass die Richtung des elektrischen Feldes im Wesentlichen parallel zur Implantatoberfläche verläuft.

11. Verfahren nach einem der Ansprüche 8 bis 10, wobei das Implantat drahtförmig ist.

12. Verfahren nach einem der Ansprüche 8 bis 11, wobei die Erwärmung des Implantates (4) und/oder des dieses umgebenden Mediums durch numerische Simulation der Ausbreitung der elektromagnetischen Strahlung in dem Testvolumen und Berechnung der Absorption der elektromagnetischen Strahlung in dem oder am Implantat erfasst wird.

**Claims**

1. Device for testing the MR-safety of an implant (4), comprising a phantom (1) that has a test volume filled with a medium (2), wherein the test volume receives the implant (4) to be tested, at least one RF transmitter (3) that emits radio-frequency electromagnetic radiation into the test volume, and comprising means (5) for detecting the heating of the implant (4) and/or of the medium (2) surrounding said implant, wherein the RF transmitter (3) comprises an assembly of a plurality of antenna elements (6), wherein each antenna element (6) can be supplied with an RF signal having an amplitude and/or phase that can be individually specified for said antenna element (6) and wherein the antenna elements (6) are applied to a wall surrounding the test volume.

2. Device according to claim 1, wherein the antenna elements (6) are connected to a multi-channel RF transmitter amplifier, wherein the amplitude and/or phase can be individually adjusted in each channel.

3. Device according to claim 1 or 2, wherein the antenna elements (6) are microstrips which extend in parallel with at least one surface of the test volume.

4. Device according to claim 3, wherein the antenna elements (6) are mutually parallel, straight microstrips.

5. Device according to any of claims 1 to 6, wherein test volume has a cuboid geometry, wherein the antenna elements (6) formed as microstrips extend substantially transversely to the longitudinal extension of the cuboid.

6. Device according to any of claims 1 to 5, wherein the medium corresponds to biological tissue in terms of electric permittivity and electrical conductivity.

7. Device according to any of claims 1 to 6, wherein the medium is a liquid or a gel or air.

8. Method for testing the MR-safety of an implant (4), wherein a device according to any of claims 1 to 7 is used and wherein the test volume that is filled with a medium (2) and that receives the implant (4) to be tested is subjected to radio-frequency electromagnetic radiation by means of at least one RF transmitter (3), wherein the heating of the implant (4) and/or of the medium (2) surrounding said implant (4) is detected.

9. Method according to claim 8, wherein each antenna element (6) can be supplied with an RF signal having an amplitude and/or phase that can be individually specified for said antenna element (6), such that an electromagnetic field is generated at the location of the implant (4) that corresponds to the electromagnetic field that is generated in the body of a patient during an MR examination.

10. Method according to claim 8 or 9, wherein the electromagnetic radiation is applied in such a way that the direction of the electrical field extends so as to be substantially in parallel with the implant surface.

11. Method according to any of claims 8 to 10, wherein the implant is wire-like.

12. Method according to any of claims 8 to 11, wherein the heating of the implant (4) and/or of the medium surrounding said implant is detected by means of a numerical simulation of the propagation of the electromagnetic radiation in the test volume and by means of calculating the absorption of the electromagnetic radiation in or at the implant.

**Revendications**

1. Dispositif pour tester la sécurité à la résonance magnétique d'un implant (4), avec un fantôme (1), qui comprend un volume de test rempli d'un milieu (2), dans lequel le volume de test loge l'implant (4) à tester, au moins un dispositif d'émission HF (3), qui émet un rayonnement électromagnétique haute fréquence à l'intérieur du volume du test, et avec des moyens (5) pour détecter réchauffement de l'implant (4) et/ou du milieu (2) entourant celui-ci, dans lequel le dispositif d'émission HF (3) comprend un ensemble composé de plusieurs éléments d'antenne (6), dans lequel chaque élément d'antenne (6) peut être soumis à l'action d'un signal HF avec une amplitude et/ou une phase pouvant être spécifiées individuellement pour ledit élément d'antenne (6) et dans lequel les éléments d'antenne (6) sont appliqués sur une paroi délimitant le volume de test.

2. Dispositif selon la revendication 1, dans lequel les éléments d'antenne (6) sont reliés à un amplificateur d'émission HF à canaux multiples, dans lequel l'amplitude et/ou la phase peuvent être réglées individuellement dans chaque canal.

3. Dispositif selon la revendication 1 ou 2, dans lequel les éléments d'antenne (6) sont des lignes en bande qui s'étendent de manière parallèle par rapport à au moins une surface du volume de test.

4. Dispositif selon la revendication 4, dans lequel les éléments d'antenne (6) sont des lignes en bande rectilignes s'étendant de manière parallèle les unes par rapport aux autres.

5. Dispositif selon l'une quelconque des revendications 1 à 4, dans lequel le volume de test présente une géométrie cuboïde, dans lequel les éléments d'antenne (6) réalisés en tant que lignes en bande s'étendent sensiblement de manière transversale par rapport à l'extension longitudinale du cube.

6. Dispositif selon l'une quelconque des revendications 1 à 5, dans lequel le milieu correspond à un tissu biologique quant à la permittivité et à la conductivité électriques.

7. Dispositif selon l'une quelconque des revendications 1 à 6, dans lequel le milieu est un liquide ou un gel ou de l'air.

8. Procédé pour tester la sécurité à la résonance magnétique d'un implant (4), où un dispositif selon l'une quelconque des revendications 1 à 7 est utilisé, dans lequel le volume de test rempli avec un milieu (2) loge l'implant (4) à tester et est soumis à l'action d'un rayonnement électromagnétique haute fréquence au moyen du dispositif d'émission HF (3), dans lequel réchauffement de l'implant (4) et/ou du milieu (2) entourant celui-ci est détecté.

9. Procédé selon la revendication 8, dans lequel chaque élément d'antenne (6) est soumis à l'action d'un signal HF avec une amplitude et/ou une phase spécifiées individuellement pour ledit élément d'antenne (6), de telle manière qu'est généré à l'emplacement de l'implant (4) un champ électromagnétique, qui correspond au champ électromagnétiquement qui précisément est généré dans le corps d'un patient au cours d'un examen à résonance magnétique.

10. Procédé selon l'une quelconque des revendications 8 ou 9, dans lequel l'application avec un rayonnement électromagnétique est effectuée de telle manière que la direction du champ électrique s'étend sensiblement de manière parallèle par rapport à la surface d'implant.

11. Procédé selon l'une quelconque des revendications 8 à 10, dans lequel l'implant est en forme de fil métallique.

12. Procédé selon l'une quelconque des revendications 8 à 11, dans lequel l'échauffement de l'implant (4) et/ou du milieu entourant celui-ci est détecté par simulation numérique de la propagation du rayonnement électromagnétique dans le volume de test et par le calcul de l'absorption du rayonnement électromagnétique dans ou sur l'implant.

**Fig. 1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• US 2010253338 A1

**In der Beschreibung aufgeführte Nicht-Patentliteratur**

• **SUKHOON OH.** Experimental and numerical Assessment of MRI-Induced Temperature Change and SAR Distributions in Phantoms and In Vivo. *Magnetic Recsonance in Medicine,* 2010, vol. 63, 218-223

• **Z. CHEN et al.** *IEEE Transactions on Microwave Theory and Techniques,* vol. 64 (3), 972-981